# EUROPEAN PATENT APPLICATION

(11) **EP 3 252 149 A1**
(43) Date of publication of application: **06.12.2017**
(21) Application number: 15873182.8
(22) Date of filing: 24.12.2015
(51) Int. Cl.: C12N 5/077, C12N 15/09, C12Q 1/06

(54) **NOVEL MATURE CARDIAC MUSCLE CELL MARKER**

(30) Priority: 25.12.2014 JP 2014262906
(71) Applicant: Kyoto University, Kyoto-shi, Kyoto 606-8501 (JP)
(72) Inventor: YOSHIDA, Yoshinori, Kyoto-shi Kyoto 606-8501 (JP); FUNAKOSHI, Shunsuke, Kyoto-shi Kyoto 606-8501 (JP)
(74) Representative: Hoffmann Eitle
(86) International application number: PCT/JP2015/086044
(87) International publication number: WO 2016/104614

(57) **Abstract**

From a cell population containing cardiomyocytes, the cardiomyocytes are extracted using a novel marker(s) specific to cardiomyocytes, that is, at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.

## Description

### TECHNICAL FIELD

The present invention relates to a method for producing cardiomyocytes using a novel cardiomyocyte marker(s). The present invention also relates to a method for testing toxicity of a drug using cardiomyocytes prepared by this method. The present invention also relates to a kit for extraction of cardiomyocytes, which kit contains a reagent for detection of the cardiomyocyte marker(s).

### BACKGROUND ART

Drug toxicity tests and cardiac disease model studies using cardiomyocytes require a large amount of mature cardiomyocytes that sufficiently mimic cardiomyocytes in the living body. Cardiomyocytes lose their division potential at the same time as the birth, and regeneration of those cells is very difficult. Because of such properties, a number of studies have been carried out for inducing differentiation of pluripotent stem cells into cardiomyocytes in order to obtain a large amount of cardiomyocytes (Patent Document 1, Patent Document 2, Non-patent Document 1, Non-patent Document 2, and Non-patent Document 3).

However, in general, it is said that cardiomyocytes derived from human pluripotent stem cells stay in an immature stage similar to fetal cardiomyocytes, and that their ion channel function is insufficient compared to adult cardiomyocytes. Thus, for the purpose of screening of drug toxicity and therapeutic agents in relation to ion channels, mature cardiomyocytes need to be used.

As described above, various methods for induction of cardiomyocytes from pluripotent stem cells have been reported. However, induction of cardiomyocytes composed only of mature cardiomyocytes has not been proposed, and the risk of inclusion of immature cardiomyocytes cannot be eliminated.

On the other hand, as indices for judging whether cells obtained by differentiation induction are cardiomyocytes or not, a plurality of marker genes have been identified so far. For example, CD166 (ALCM) (Non-patent Document 4), N-cadherin (Patent Document 3 or Non-patent Document 5), VCAM1 (Patent Document 4), and the like have been reported as surface markers for cardiomyocytes, but there is no report on marker genes specific to mature cardiomyocytes.

### PRIOR ART DOCUMENTS

### [Patent Documents]

Patent Document 1: WO 2007/002136
Patent Document 2: WO 2009/118928
Patent Document 3: JP 2010-158206 A
Patent Document 4: WO 2012/133954

### [Non-patent Documents]

Non-patent Document 1: Yan P, et al, Biochem Biophys Res Commun. 379: 115-20 (2009)
Non-patent Document 2: Laflamme MA, et al, Nat Biotechnol, 25: 1015-1024 (2007)
Non-patent Document 3: Yang L et al, Nature, 453: 524-528 (2008)
Non-patent Document 4: Rust W, et al, Regen Med. 4, 225-37 (2009)
Non-patent Document 5: Honda M, et al, Biochem Biophys Res Commun. 29, 351, 877-82 (2006)

### SUMMARY OF THE INVENTION

An object of the present invention is to extract cardiomyocytes from a cell population containing the cardiomyocytes. Accordingly, an object of the present invention is to provide markers specific to cardiomyocytes.

As a result of intensive study to solve the above problem, the present inventors found that cardiomyocytes can be obtained at high frequency from a cell population containing cardiomyocytes using, as an index, positivity of at least one selected from the group consisting of CORIN, NCAM1 (Neural cell adhesion molecule 1), CRYAB (crystallin, alpha B), HBEGF (heparin binding-epidermal growth factor-like growth factor), DMD (dystrophin), ATPIF1 (ATPase inhibitory factor 1), CAV2 (Caveolin-2), ITGAV (Integrin alpha-V), DCBLD2 (discoidin, CUB and LCCL domain containing 2), CLIC4 (Chloride intracellular channel 4), BMPR2 (Bone morphogenetic protein receptor type II), CTSB (cathepsin B), TMEM123 (transmembrane protein 123), USP14 (Ubiquitin carboxyl-terminal hydrolase 14), and MIR761. Based on the above findings, the present inventors succeeded in isolation and purification of cardiomyocytes by using, as an index, positivity of at least one selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761, thereby completed the present invention.

The present invention provides the followings:
[1] A method for producing cardiomyocytes, comprising extracting mature cardiomyocytes from a cell population containing cardiomyocytes using, as an index, positivity of at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.
[2] The method according to [1], wherein said marker is CORIN.
[3] The method according to [1] or [2], wherein cells in which the expression level(s) of said marker(s) is/are ranked in the top 50% are extracted as mature cardiomyocytes from the cells contained in the cell population containing cardiomyocytes.
[4] The method according to [3], wherein cells in which the expression level(s) of said marker(s) is/are ranked in the top 33% are extracted as mature cardiomyocytes from the cells contained in the cell population containing cardiomyocytes.
[5] The method according to any one of [1] to [4], wherein said cardiomyocytes are human cardiomyocytes.
[6] The method according to any one of [1] to [5], wherein said cell population containing cardiomyocytes is a cell population containing cardiomyocytes obtained by differentiation induction from pluripotent stem cells.
[7] The method according to [6], wherein said pluripotent stem cells are ES cells or iPS cells.
[8] The method according to [6] or [7], wherein said differentiation induction into cardiomyocytes comprises forming an embryoid body/bodies.
[9] The method according to [8], wherein said differentiation induction into cardiomyocytes comprises culturing an embryoid body/bodies in a medium containing a cytokine.
[10] The method according to [9], wherein said cytokine is at least one cytokine selected from the group consisting of activin A, BMP4, b-FGF, VEGF, and Wnt inhibitors.
[11] The method according to [10], wherein said differentiation induction into cardiomyocytes comprises:
   (1) forming an embryoid body/bodies from pluripotent stem cells;
   (2) culturing the embryoid body/bodies obtained in Step (1) in a medium containing activin A, BMP4, and bFGF;
   (3) dissociating the embryoid body/bodies obtained in Step (2);
   (4) culturing the cells obtained in Step (3) in a medium containing VEGF and a Wnt inhibitor, to allow reaggregation of the cells into an embryoid body/bodies; and
   (5) culturing the embryoid body/bodies obtained in Step (4) in a medium containing VEGF and bFGF.
[12] The method according to [11], wherein said Wnt inhibitor is IWP-3 or IWP-4.
[13] The method according to[11] or [12], wherein Step (5) in the differentiation induction into cardiomyocytes includes a culture period when the cells are cultured in a medium which contains lactic acid but does not contain glucose.
[14] A method for testing cardiotoxicity of a drug candidate substance, comprising:
   (A) bringing mature cardiomyocytes prepared by the method according to any one of [1] to [13] into contact with a candidate substance; and
   (B) identifying said candidate substance as a drug having no cardiotoxicity in a case where the cardiomyocytes maintains a normal function after the contact in said step (A).
[15] The method according to [14], wherein said normal function of the cardiomyocytes is an electrophysiologically normal function.
[16] A mature cardiomyocyte extraction kit comprising a reagent which detects at least one selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.
[17] The kit according to [16], comprising a reagent for detection of CORIN.
[18] The kit according to [16] or [17], wherein said cardiomyocyte is a human cardiomyocyte.
[19] A method for producing cardiomyocytes, comprising:
   obtaining a cell population containing cardiomyocytes from pluripotent stem cells by the following steps (1) to (5):
      (1) forming an embryoid body/bodies from pluripotent stem cells;
      (2) culturing the embryoid body/bodies obtained in Step (1) in a medium containing activin A, BMP4, and bFGF;
      (3) dissociating the embryoid body/bodies obtained in Step (2);
      (4) culturing the cells obtained in Step (3) in a medium containing VEGF and a Wnt inhibitor, to allow reaggregation of the cells into an embryoid body/bodies; and,
      (5) culturing the embryoid body/bodies obtained in Step (4) in a medium containing VEGF and bFGF; and
   extracting mature cardiomyocytes from the obtained cell population containing cardiomyocytes using, as an index, positivity of at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.

By the present invention, mature cardiomyocytes having high similarly to cardiomyocytes present in the living body can be provided. Thus, the present invention is useful for drug toxicity tests and cardiac disease model studies with higher accuracy.

### BRIEF DESCRIPTION OF THE DRAWINGS

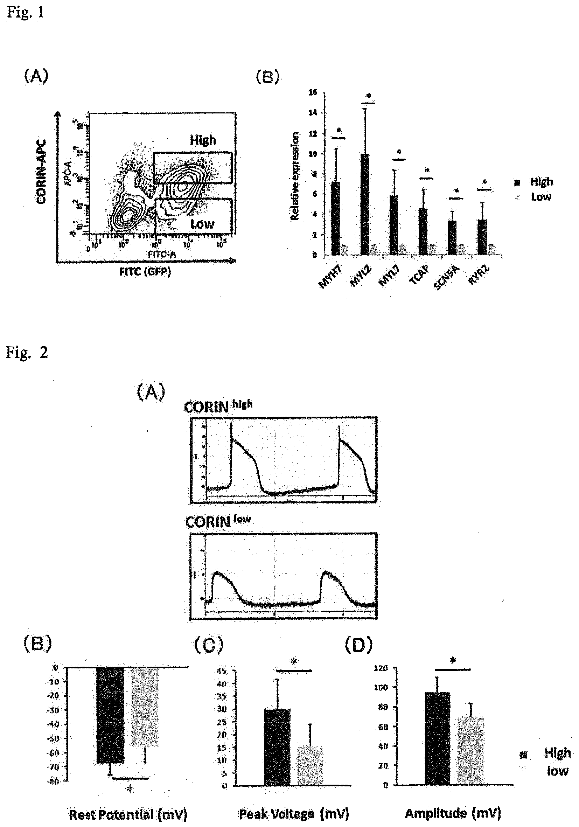

Fig. 1 shows results obtained by comparing the maturity of cardiomyocytes between cells showing high expression of CORIN and cells showing low expression of CORIN. (A) shows results of FACS analysis in which cells on Day 30 after differentiation induction into cardiomyocytes were subjected to sorting with GFP and CORIN. (B) shows results of comparison of the relative expression levels of cardiomyocyte markers (MYH7, MYL2, MYL7, TCAP, SCN5A, and RYR2). In this figure, "High" represents cells showing high expression of both GFP and CORIN, and "Low" represents cells showing high expression of GFP and low expression of CORIN.
Fig. 2 shows results of tests on cardiomyocyte functions by electrophysiological methods. (A) shows the result of electrophysiological comparison between cells showing high expression of CORIN and cells showing low expression of CORIN among cardiomyocytes on Day 30 after differentiation induction into cardiomyocytes. (B) shows the result of comparison of the resting membrane potential between the same groups of cells as in (A). (C) shows the result of comparison of the peak voltage between the same groups of cells as in (A). (D) shows the result of comparison of the amplitude between the same groups of cells as in (A).

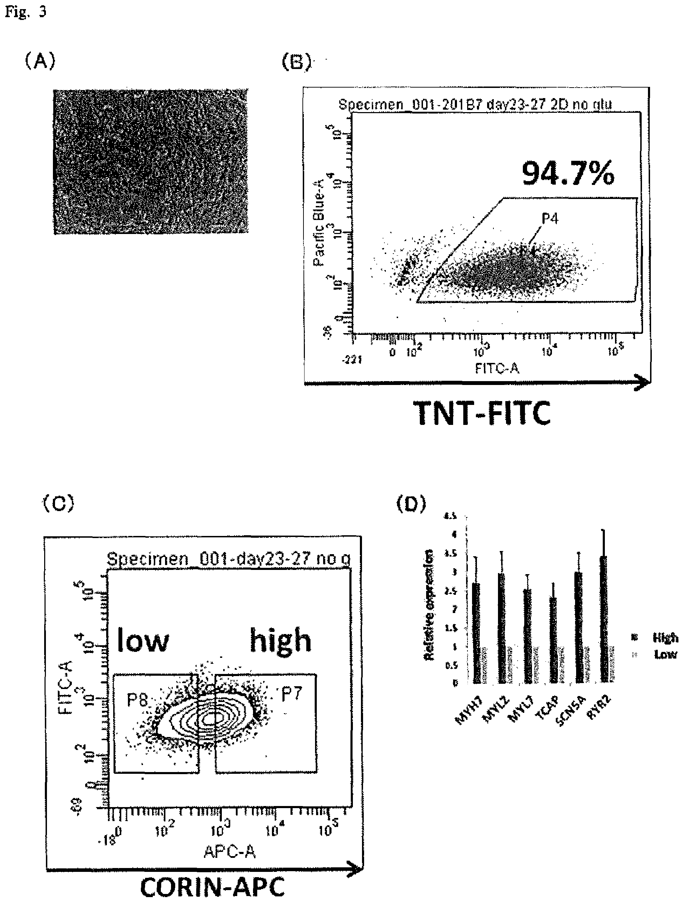

Fig. 3 shows results of comparison of the maturity of cardiomyocytes between cells showing high expression of CORIN and cells showing low expression of CORIN. (A) shows a photograph showing the morphology of cells on Day 30 after differentiation induction into cardiomyocytes. The cells were placed under conditions where glucose is absent and lactic acid is present from Day 23 to Day 27, to induce cell death of non-cardiomyocytes. (B) shows the result of FACS analysis in which cells on Day 30 after differentiation induction into cardiomyocytes were subjected to sorting with a known cardiomyocyte marker (TNT). (C) shows the result of FACS analysis in which the cells positive for the cardiomyocyte marker (TNT) sorted in (B) were subjected to sorting with CORIN. (D) shows the result of comparison of the relative expression levels of cardiomyocyte markers (MYH7, MYL2, MYL7, TCAP, SCN5A, and RYR2). In this figure, "High" represents cells showing high expression of both the known cardiomyocyte marker (TNT) and CORIN, and "Low" represents cells showing high expression of the known cardiomyocyte marker (TNT) and low expression of CORIN.

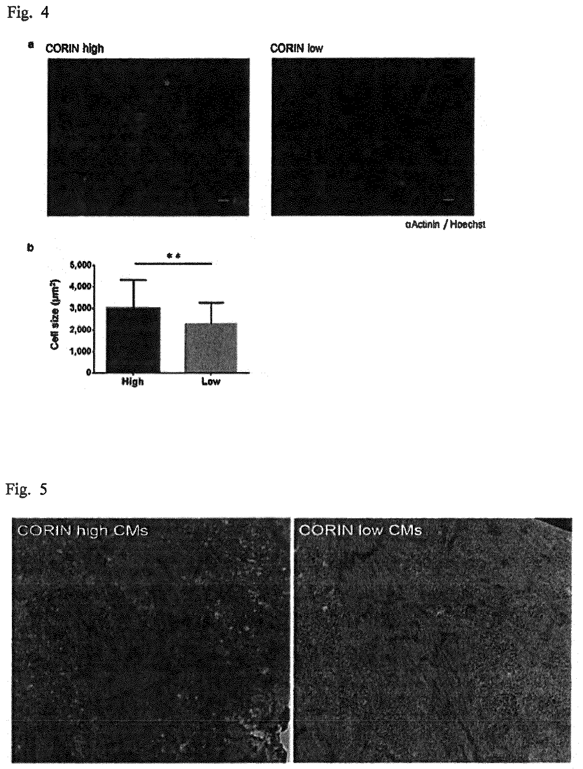

Fig. 4 shows micrographs showing the cell sizes in the CORIN-High group and the CORIN-Low group (a), and a graph showing the result of comparison of the cell size (b). In (a), the red color represents actinin; the blue color represents Hoechst 33342; and the scale bar represents 20 µm. In (b), each value is expressed as mean ± SD, and "**" represents p<0.01 in the unpaired t-test.
Fig. 5 shows electron micrographs showing the cardiac muscle sarcomere structures in the CORIN-High group (left) and the CORIN-Low group (right).

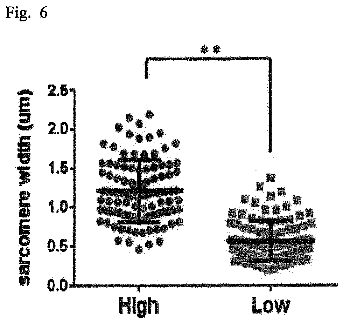

Fig. 6 shows a graph for comparison of the sarcomere width between the CORIN-High group (left) and the CORIN-Low group (right), which width was determined based on the electron micrograph data in Fig. 5. The values are expressed as mean ± SD, and "**" represents p<0.01 in the unpaired t-test.

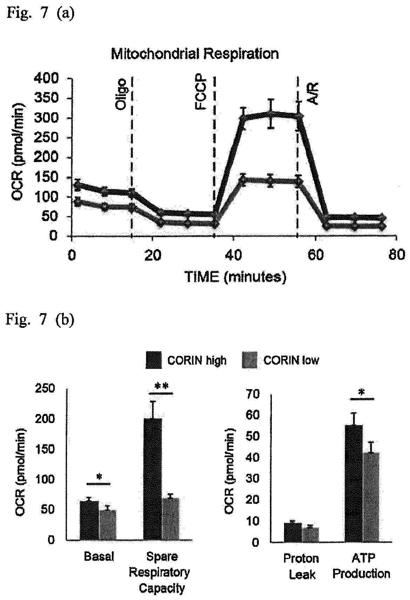

Fig. 7 shows diagrams showing evaluation of mitochondrial respiration in the CORIN-High group (black) and the CORIN-Low group (gray). (a) shows a graph showing changes in the oxygen consumption rate (OCR) with time as observed using mitochondrial function inhibitors (Oligo: oligomycin, FCCP: carbonyl cyanide-4-phenylhydrazone, A/R: antimycine + rotenone). The data are shown as mean ± SD. (b) shows a graph showing values of the basal respiration, spare respiratory capacity, proton leak, and ATP-linked respiration calculated from the data in (a). The values are expressed as mean ± SD, and "*" and "**" represent p<0.05 and p<0.001, respectively, in the unpaired t-test.

### EMBODIMENTS FOR CARRYING OUT THE INVENTION

The present invention is described below in detail.

As described above, the present invention relates to a method for producing a cardiomyocyte(s), which method comprises the step of extracting cardiomyocytes from a cell population containing cardiomyocytes using, as an index, positivity of at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761; a method for testing toxicity of a drug using cardiomyocytes prepared by this method; and a kit for extraction of cardiomyocytes, which kit contains a reagent for detection of the cardiomyocyte marker(s).

In the present invention, "cardiomyocyte" means a cell expressing at least one marker gene selected from the group consisting of cardiac troponin (cTNT), αMHC (α myosin heavy chain), and MYH6. Examples of cTNT include NCBI accession number NM_000364 in a case of human, and NM_001130174 in a case of mouse. Examples of αMHC include NCBI accession number NM_002471 in a case of human, and NM_001164171 in a case of mouse. Examples of MYH6 include NCBI accession number NM_002471 in a case of human, and NM_001164171 in a case of mouse.

In the present invention, "mature cardiomyocyte" means a cardiomyocyte which is functionally and morphologically similar to an adult cardiomyocyte that is present in the living body. That is, the mature cardiomyocyte in the present invention is a cardiomyocyte having sufficiently developed ion channel function relative to that of a fetal cardiomyocyte, which is in an immature stage, and examples of the mature cardiomyocyte include cells whose resting membrane potential, peak voltage, amplitude, and/or the like is/are similar to those of adult cardiomyocytes. The mature cardiomyocyte in the present invention is characterized by a resting membrane potential of, for example, not more than -55 mV, preferably not more than -60 mV, more preferably not more than -65 mV; a peak voltage of not less than 15 mV, preferably not less than 20 mV, more preferably not less than 25 mV; and an amplitude of not less than 70 mV, preferably not less than 80 mV, more preferably not less than 90 mV.

"Mature cardiomyocyte" is characterized also by expression of one or more markers selected from MYH7, MYL2, MYL7, TCAP, SCN5A, and RYR2.

Examples of the sequences of the genes encoding the novel mature cardiomyocyte markers in the present invention are shown in Table 1. However, the sequences of the markers are not limited to the following sequences as long as they are expressed in the cells to be extracted.

**[Table 1]**

| Gene name | NCBI Accession No. | |
|---|---|---|
| | Mouse | Human |
| CORIN | NM_ 001122756 | NM_001 278585 |
| NCAM1 | NM_ 001081445 | NM_ 000615 |
| CRYAB | NM_ 001289782 | NM_ 001289807 |
| HBEGF | NM_ 010415 | NM_ 001945 |
| DMD | NM_ 007868 | NM_ 000109 |
| ATPIF1 | NM_ 007512 | NM_ 016311 |
| CAV2 | NM_ 001277756 | NM_ 001206747 |
| ITGAV | NM_ 008402 | NM_ 001144999 |
| DCBLD2 | NM_ 028523 | NM_ 080927 |
| CLIC4 | NM_ 013885 | NM_ 013943 |
| BMPR2 | NM_ 007561 | NM_001204 |
| CTSB | NM_ 007798 | NM_001908 |
| TMEM123 | NM_ 133739 | NM_ 052932 |
| USP14 | NM_001038589 | NM_001037334 |
| MIR761 | NR_030432 | NR_031580 |

When mature cardiomyocytes are extracted using the novel mature cardiomyocyte marker(s) in the present invention, expression of each marker protein may be used as an index, and/or expression of the gene encoding each marker protein (expression of mRNA) may be used as an index. In cases where each gene has an isoform due to alternative splicing, the isoform is also included in the scope of the gene.

In the present invention, the origin of the "cell population containing cardiomyocytes" is not limited as long as it is a cell population containing mature cardiomyocytes. Examples of the cell population include cell populations contained in peripheral blood, heart, myeloid tissue, adipose tissue, skeletal muscle tissue, amniotic tissue, placental tissue, umbilical cord blood, or the like obtained by an arbitrary method, and cell populations containing cardiomyocytes obtained by differentiation induction from pluripotent stem cells.

In the present invention, "extraction of mature cardiomyocytes" means increasing of the ratio of mature cardiomyocytes relative to that before the extraction. The term preferably means that mature cardiomyocytes are concentrated to a ratio of not less than 50%, 60%, 70%, 80%, or 90%. The term more preferably means that cells composed of mature cardiomyocytes at a ratio of 100% are obtained.

In the present invention, when mature cardiomyocytes are extracted from a cell population containing cardiomyocytes using as an index/indices CORIN, NCAM1, CRYAB, HBEGF, DMD, ATPIF1, CAV2, ITGAV, DCBLD2, CLIC4, BMPR2, CTSB, TMEM123, USP14, and/or MIR761, these genes, or the proteins encoded by these genes may be used individually or in an arbitrary combination. In cases where CORIN, NCAM1, CRYAB, HBEGF, DMD, ATPIF1, CAV2, ITGAV, DCBLD2, CLIC4, BMPR2, CTSB, TMEM123, USP14, and/or MIR761 are used in an arbitrary combination, the concentration ratio of the cardiomyocytes is preferably increased relative to those in cases of single use. In particular, NCAM1 is preferably used in combination with another cardiomyocyte marker(s) described above rather than being used alone.

### <Pluripotent Stem Cells>

The pluripotent stem cells which may be used for obtaining the cell population containing cardiomyocytes in the present invention are stem cells having pluripotency which enables the cells to differentiate into any cells present in the living body, which stem cells also have growth ability. Examples of the pluripotent stem cells include, but are not limited to, embryonic stem (ES) cells, germline stem cells ("GS cells"), embryonic germ cells ("EG cells"), induced pluripotent stem (iPS) cells, and embryonic stem cells derived from a cloned embryo obtained by nuclear transfer (ntES cells). The pluripotent stem cells are preferably ES cells, iPS cells, or ntES cells.

### (A) Embryonic Stem Cells

ES cells are stem cells established from the inner cell mass of an early embryo (for example, blastocyst) of a mammal such as human or mouse, which cells have pluripotency and growth ability by self-renewal.

ES cells are embryo-derived stem cells originated from the inner cell mass of a blastocyst, which is the embryo formed following the 8-cell stage and the morula stage of a fertilized egg. ES cells have ability to differentiate into any cells constituting an adult, that is, the so-called pluripotency of differentiation, and growth ability by self-renewal. ES cells were discovered in mouse in 1981 (M. J. Evans and M. H. Kaufman (1981), Nature 292: 154-156), and this was followed by establishment of ES cell lines of primates such as human and monkey (J. A. Thomson et al. (1998), Science 282: 1145-1147; J. A. Thomson et al. (1995), Proc. Natl. Acad. Sci. USA, 92: 7844-7848; J. A. Thomson et al. (1996), Biol. Reprod., 55: 254-259; J. A. Thomson and V. S. Marshall (1998), Curr. Top. Dev. Biol., 38: 133-165).

ES cells can be established by removing the inner cell mass from the blastocyst of a fertilized egg of a subject animal, followed by culturing the inner cell mass on feeder fibroblasts. The cells can be maintained by subculture using a medium supplemented with a substance(s) such as leukemia inhibitory factor (LIF) and/or basic fibroblast growth factor (bFGF). Methods of establishment and maintenance of human and monkey ES cells are described in, for example, US 5,843,780 B; Thomson JA, et al. (1995), Proc Natl. Acad. Sci. USA. 92: 7844-7848; Thomson JA, et al. (1998), Science. 282: 1145-1147; H. Suemori et al. (2006), Biochem. Biophys. Res. Commun., 345: 926-932; M. Ueno et al. (2006), Proc. Natl. Acad. Sci. USA, 103: 9554-9559; H. Suemori et al. (2001), Dev. Dyn., 222: 273-279; H. Kawasaki et al. (2002), Proc. Natl. Acad. Sci. USA, 99: 1580-1585; and Klimanskaya I, et al. (2006), Nature. 444: 481-485.

In terms of the medium for preparation of ES cells, human ES cells can be maintained, for example, using DMEM/F-12 medium supplemented with 0.1 mM 2-mercaptoethanol, 0.1 mM non-essential amino acids, 2 mM L-glutamic acid, 20% KSR and 4 ng/ml bFGF at 37°C under a moist atmosphere of 2% CO₂/98% air (O. Fumitaka et al. (2008), Nat. Biotechnol., 26:215-224). ES cells need to be subcultured every 3 to 4 days, and the subculture can be carried out using, for example, 0.25% trypsin and 0.1 mg/ml collagenase IV in PBS supplemented with 1 mM CaCl₂ and 20% KSR.

Selection of ES cells can be generally carried out by the Real-Time PCR method using as an index/indices expression of a gene marker(s) such as alkaline phosphatase, Oct-3/4, and/or Nanog. In particular, for selection of human ES cells, expression of a gene marker(s) such as OCT-3/4, NANOG, and/or ECAD can be used as an index/indices (E. Kroon et al. (2008), Nat. Biotechnol., 26: 443-452).

Examples of available human ES cell lines include WA01(H1) and WA09(H9), which can be obtained from WiCell Research Institute; and KhES-1, KhES-2, and KhES-3, which can be obtained from Institute for Frontier Medical Sciences, Kyoto University (Kyoto, Japan).

### (B) Germline Stem Cells

Germline stem cells are pluripotent stem cells derived from testis, and play a role as the origin for spermatogenesis. Similarly to ES cells, these cells can be induced to differentiate into various series of cells, and, for example, have a property to enable preparation of a chimeric mouse by transplantation of the cells to a mouse blastocyst (M. Kanatsu-Shinohara et al. (2003) Biol. Reprod., 69: 612-616; K. Shinohara et al. (2004), Cell, 119: 1001-1012). Germline stem cells are capable of self-renewal in a medium containing glial cell line-derived neurotrophic factor (GDNF), and, by repeating their subculture under the same culture conditions as those for ES cells, germline stem cells can be obtained (Masanori Takehashi et al. (2008), Experimental Medicine, 26(5) (extra edition): 41-46, Yodosha (Tokyo, Japan)).

### (C) Embryonic Germ Cells

Embryonic germ cells are established from fetal primordial germ cells and have pluripotency similarly to ES cells. They can be established by culturing primordial germ cells in the presence of substances such as LIF, bFGF, and stem cell factor (Y. Matsui et al. (1992), Cell, 70: 841-847; J. L. Resnick et al. (1992), Nature, 359: 550-551).

### (D) Induced Pluripotent Stem Cells

Induced pluripotent stem (iPS) cells can be prepared by introducing particular reprogramming factors to somatic cells, which reprogramming factors are in the form of DNA or protein. iPS cells are somatic cell-derived artificial stem cells having properties almost equivalent to those of ES cells, such as pluripotency of differentiation and growth ability by self-renewal (K. Takahashi and S. Yamanaka (2006) Cell, 126: 663-676; K. Takahashi et al. (2007), Cell, 131: 861-872; J. Yu et al. (2007), Science, 318: 1917-1920; Nakagawa, M. et al., Nat. Biotechnol. 26: 101-106 (2008); WO 2007/069666). The reprogramming factors may be constituted by genes or gene products thereof, or non-coding RNAs, which are expressed specifically in ES cells; or genes or gene products thereof, non-coding RNAs, or low molecular weight compounds, which play important roles in maintenance of the undifferentiated state of ES cells. Examples of the genes included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2 and Tbx3, and these reprogramming factors may be used either individually or in combination. Examples of the combination of the reprogramming factors include those described in WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26: 2467-2474; Huangfu D, et al. (2008), Nat Biotechnol. 26: 1269-1275; Shi Y, et al. (2008), Cell Stem Cell, 3, 568-574; Zhao Y, et al. (2008), Cell Stem Cell, 3: 475-479; Marson A, (2008), Cell Stem Cell, 3, 132-135; Feng B, et al. (2009), Nat Cell Biol. 11: 197-203; R. L. Judson et al., (2009), Nat. Biotech., 27: 459-461; Lyssiotis CA, et al. (2009), Proc Natl Acad Sci USA. 106:8912-8917; Kim JB, et al. (2009), Nature. 461: 649-643; Ichida JK, et al. (2009), Cell Stem Cell. 5: 491-503; Heng JC, et al. (2010), Cell Stem Cell. 6: 167-74; Han J, et al. (2010), Nature. 463: 1096-100; and Mali P, et al. (2010), Stem Cells. 28: 713-720.

Examples of the above-described reprogramming factors also include histone deacetylase (HDAC) inhibitors [for example, low molecular weight inhibitors such as valproic acid (VPA), trichostatin A, sodium butyrate, MC 1293, and M344; and nucleic acid-type expression inhibitors such as siRNAs and shRNAs against HDAC (e.g., HDAC1 siRNA Smartpool (registered trademark) (Millipore) and HuSH 29mer shRNA Constructs against HDAC1 (OriGene))], MEK inhibitors (for example, PD184352, PD98059, U0126, SL327, and PD0325901), Glycogen synthase kinase-3 inhibitors (for example, Bio and CHIR99021), DNA methyltransferase inhibitors (for example, 5'-azacytidine), histone methyltransferase inhibitors (for example, low molecular weight inhibitors such as BIX-01294; and nucleic acid-type expression inhibitors such as siRNAs and shRNAs against Suv39hl, Suv39h2, SetDB1, and G9a), L-channel calcium agonists (for example, Bayk8644), butyric acid, TGFβ inhibitors or ALK5 inhibitors (for example, LY364947, SB431542, 616453 and A-83-01), p53 inhibitors (for example, siRNAs and shRNAs against p53), ARID3A inhibitors (for example, siRNAs and shRNAs against ARID3A), miRNAs such as miR-291-3p, miR-294, miR-295, and mir-302, Wnt Signaling (for example, soluble Wnt3a), neuropeptide Y, prostaglandins (for example, prostaglandin E2 and prostaglandin J2), hTERT, SV40LT, UTF1, IRX6, GLIS1, PITX2, and DMRTB1, which are factors employed for enhancing the establishment efficiency, and, in the present description, these factors employed for the purpose of enhancement of the establishment efficiency are not particularly distinguished from reprogramming factors.

In cases where the reprogramming factors are in the form of protein, the reprogramming factors may be introduced into somatic cells by a method such as lipofection, fusion with a cell membrane-permeable peptide (e.g., HIV-derived TAT or polyarginine), or microinjection.

In cases where the reprogramming factors are in the form of DNA, the reprogramming factors may be introduced into somatic cells by a method such as use of a vector such as a virus, plasmid, or artificial chromosome vector; lipofection; use of liposome; or microinjection. Examples of the virus vector include retrovirus vectors, lentivirus vectors (these are described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors, and Sendai virus vectors (WO 2010/008054). Examples of the artificial chromosome vector include human artificial chromosome (HAC), yeast artificial chromosome (YAC), and bacterial artificial chromosome (BAC, PAC). Examples of the plasmid which may be used include plasmids for mammalian cells (Science, 322: 949-953, 2008). The vectors may contain a regulatory sequence such as a promoter, enhancer, ribosome binding sequence, terminator, or polyadenylation site for allowing expression of each nuclear reprogramming substance; and, if necessary, a sequence of a selection marker such as a drug resistance gene (for example, kanamycin resistance gene, ampicillin resistance gene, or puromycin resistance gene), thymidine kinase gene, or diphtheria toxin gene; a gene sequence of a reporter such as the green-fluorescent protein (GFP), β-glucuronidase (GUS), or FLAG; or the like. Further, in order to remove, after introduction of the above vector into somatic cells, the genes encoding the reprogramming factors, or both the promoters and the genes encoding the reprogramming factors linked thereto, the vector may have LoxP sequences upstream and downstream of these sequences.

In cases where the reprogramming factors are in the form of RNA, each reprogramming factor may be introduced into somatic cells by a method such as lipofection or microinjection, and RNA in which 5-methylcytidine and pseudouridine (TriLink Biotechnologies) are incorporated may be used in order to suppress degradation (Warren L, (2010) Cell Stem Cell. 7:618-630).

Examples of the medium for induction of the iPS cells include DMEM, DMEM/F12, and DME media supplemented with 10 to 15% FBS (these media may further contain LIF, penicillin/streptomycin, puromycin, L-glutamine, non-essential amino acids, β-mercaptoethanol, or the like, if appropriate). Other examples of the medium for induction of the iPS cells include commercially available media [for example, a medium for culturing mouse ES cells (TX-WES medium, Thromb-X), medium for culturing primate ES cells (medium for primate ES/iPS cells, ReproCELL), and serum-free medium (mTeSR, Stemcell Technology)].

Examples of the culture method include a method wherein somatic cells and reprogramming factors are brought into contact with each other at 37°C in the presence of 5% CO₂ on DMEM or DMEM/F12 medium supplemented with 10% FBS, and the cells are then cultured for about 4 to 7 days, followed by plating the cells on feeder cells (e.g., mitomycin C-treated STO cells or SNL cells) and starting culture in a bFGF-containing medium for culturing primate ES cells about 10 days after the contact between the somatic cells and the reprogramming factors, thereby allowing iPS-like colonies to appear about 30 to about 45 days after the contact, or later.

Alternatively, the cells may be cultured at 37°C in the presence of 5% CO₂ on feeder cells (e.g., mitomycin C-treated STO cells or SNL cells) in DMEM medium supplemented with 10% FBS (this medium may further contain LIF, penicillin/streptomycin, puromycin, L-glutamine, non-essential amino acids, β-mercaptoethanol, or the like, as appropriate) for about 25 to about 30 days or longer, to allow ES-like colonies to appear. Preferred examples of the culture method include a method wherein the somatic cells themselves to be reprogrammed are used instead of the feeder cells (Takahashi K, et al. (2009), PLoS One. 4: e8067 or WO 2010/137746), and a method wherein an extracellular matrix (e.g., Laminin-5 (WO 2009/123349) or Matrigel (BD)) is used instead.

Other examples of the culture method include a method wherein culture is carried out using a serum-free medium (Sun N, et al. (2009), Proc Natl Acad Sci U S A. 106:15720-15725). Further, in order to enhance the establishment efficiency, iPS cells may be established under low oxygen conditions (at an oxygen concentration of 0.1% to 15%) (Yoshida Y, et al. (2009), Cell Stem Cell. 5: 237-241 or WO 2010/013845).

During the culture, the medium is replaced with a fresh medium once every day from Day 2 of the culture. The number of the somatic cells used for nuclear reprogramming is not limited, and usually within the range of about 5×10³ to about 5×10⁶ cells per 100-cm² area on the culture dish.

iPS cells can be selected based on the shapes of the colonies formed. In cases where a drug resistance gene expressed in conjunction with a gene that is expressed upon reprogramming of a somatic cell (e.g., Oct3/4 or Nanog) is introduced as a marker gene, established iPS cells can be selected by culturing the cells in a medium containing the corresponding drug (selection medium). iPS cells can be selected by observation under a fluorescence microscope in cases where the marker gene is the gene of a fluorescent protein, by adding a luminescent substrate in cases where the marker gene is the gene of luciferase, or by adding a coloring substrate in cases where the marker gene is the gene of a coloring enzyme.

The term "somatic cells" used in the present description means any animal cells (preferably cells of mammals including human) excluding germ-line cells and totipotent cells such as eggs, oocytes, and ES cells. Examples of the somatic cells include, but are not limited to, any of fetal somatic cells, neonatal somatic cells, and mature, healthy or diseased somatic cells, as well as any of primary cultured cells, subcultured cells, and established cell lines. Specific examples of the somatic cells include (1) tissue stem cells (somatic stem cells) such as neural stem cells, hematopoietic stem cells, mesenchymal stem cells, and dental pulp stem cells; (2) tissue progenitor cells; and (3) differentiated cells such as lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells and adipocytes.

In the present invention, the mammalian individual from which somatic cells are collected is not limited, and preferably human. In cases where the obtained iPS cells are to be used for human regenerative medicine, it is especially preferred to collect somatic cells from the patient himself or another person having the same or substantially the same HLA type in view of prevention of the rejection reaction. Here, "substantially the same" HLA type means that the HLA type is matching to an extent which allows survival of transplanted cells by use of an immunosuppressant(s) and/or the like when cells obtained by differentiation induction from iPS cells derived from the somatic cells are transplanted to the patient. For example, it means that the person has the same major HLAs (for example, at the three loci HLA-A, HLA-B, and HLA-DR) as those of the patient (the same applies hereinafter). On the other hand, in cases where the cells are not administered (transplanted) to human, for example, in a method for testing toxicity of a candidate drug to cardiomyocytes, the origin of the somatic cells to be used as the source of the iPS cells is not limited. In cases where the iPS cells are used as the source of cells for screening for evaluation of drug sensitivity of a patient or the risk of production of side effects, it is preferred to collect somatic cells from the patient himself or another person who has the same genetic polymorphism(s) associated with the drug sensitivity or the side effects.

### (E) ES Cells Derived from Cloned Embryo Obtained by Nuclear Transfer

ntES cells are ES cells derived from a cloned embryo prepared by the nuclear transfer technique, and have almost the same properties as those of ES cells derived from fertilized eggs (T. Wakayama et al. (2001), Science, 292: 740-743; S. Wakayama et al. (2005), Biol. Reprod., 72: 932-936; J. Byrne et al. (2007), Nature, 450: 497-502). That is, an ntES (nuclear transfer ES) cell is an ES cell established from the inner cell mass of a blastocyst derived from a cloned embryo obtained by replacement of the nucleus of an unfertilized egg with the nucleus of a somatic cell. For preparation of an ntES cell, the combination of the nuclear transfer technique (J.B. Cibelli et al. (1998), Nature Biotechnol., 16: 642-646) and the ES cell preparation technique (described above) is employed (Sayaka Wakayama et al. (2008), Experimental Medicine 26(5) (extra edition), pp. 47-52). In nuclear transfer, reprogramming can be achieved by injecting the nucleus of a somatic cell into a mammalian enucleated unfertilized egg and culturing the resultant for several hours.

### <Method for Producing Cell Population Containing Cardiomyocytes from Pluripotent Stem Cells>

In the method for the differentiation induction into the cell population containing cardiomyocytes in the present invention, cardiomyocytes can be produced from pluripotent stem cells by, for example, a method reported by Laflamme MA et al. (Laflamme MA & Murry CE, Nature 2011, Review). Other examples of the method include, but are not limited to, a method in which cardiomyocytes are produced by formation of cell clusters (embryoid bodies) by suspension culture of induced pluripotent stem cells, a method in which cardiomyocytes are produced in the presence of a substance that suppresses BMP signaling (WO 2005/033298), a method in which cardiomyocytes are produced by addition of Activin A and BMP in this order (WO 2007/002136), a method in which cardiomyocytes are produced in the presence of a substance that promotes activation of the canonical Wnt signaling pathway (WO 2007/126077), and a method in which Flk/KDR-positive cells are isolated from induced pluripotent stem cells, followed by production of cardiomyocytes in the presence of cyclosporin A (WO 2009/118928).

In the present invention, the method of differentiation induction of pluripotent stem cells into a cell population containing cardiomyocytes is not limited, and examples of the method include the followings.

### <Step of Forming Embryoid Bodies from Pluripotent Stem Cells: Step (1)>

Preferably, in this step, pluripotent stem cells forming colonies are dissociated into single cells, and then allowed to form embryoid bodies. In the step of dissociating the pluripotent stem cells, cells adhering to each other and forming populations are dissociated (separated) into individual cells. Examples of the method for dissociating the pluripotent stem cells include a method in which the cells are mechanically dissociated, and a dissociation method in which a dissociation solution having protease activity and collagenase activity (e.g., Accutase (trademark) or Accumax (trademark)) or a dissociation solution having only collagenase activity is used. The method is preferably a method in which a dissociation solution having protease activity and collagenase activity (especially preferably Accumax) is used to dissociate the pluripotent stem cells.

In the method of the present invention, examples of the method for forming the embryoid bodies include subjecting the dissociated pluripotent stem cells to suspension culture using a culture dish whose surface is not artificially treated for the purpose of improving adhesion of cells thereto (for example, not subjected to coating treatment with Matrigel (BD), collagen, gelatin, laminin, heparan sulfate proteoglycan, or entactin) or using a culture dish which is artificially treated such that the adhesion is suppressed (for example, treated by coating with polyhydroxyethyl methacrylate (poly-HEMA)).

### <Step of Culturing Embryoid Bodies in Medium Containing Activin A, BMP4, and bFGF: Step (2)>

The medium used in this step can be prepared using, as a basal medium, a medium for animal cell culture, and adding activin A, BMP4, and bFGF thereto. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, Neurobasal Medium (Life Technologies), and StemPro34 (Invitrogen), and mixed media thereof. The medium may contain serum, or may be serum-free. If necessary, the medium may contain one or more of serum replacements such as albumin, transferrin, Knockout Serum Replacement (KSR) (serum replacement for FBS in ES cell culture), N2 supplement (Invitrogen), B27 supplement (Invitrogen), fatty acids, insulin, collagen precursor, trace elements, 2-mercaptoethanol, and 1-thiolglycerol, and may also contain one or more of substances such as lipids, amino acids, L-glutamine, Glutamax (Invitrogen), non-essential amino acids, vitamins, growth factors, low-molecular-weight compounds, antibiotics, antioxidants, pyruvic acid, buffers, and inorganic salts. A preferred basal medium is StemPro34, which contains transferrin, 1-thiolglycerol, L-glutamine, and ascorbic acid.

The concentration of activin A to be used in the present step is preferably 1 ng/ml to 100 ng/ml. Examples of the concentration of activin A include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of activin A is especially preferably 12 ng/ml.

The concentration of BMP4 to be used in the present step is preferably 1 ng/ml to 100 ng/ml. Examples of the concentration of BMP4 include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of BMP4 is especially preferably 18 ng/ml.

The concentration of bFGF to be used in the present step is preferably 1 ng/ml to 100 ng/ml. Examples of the concentration of bFGF include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of VEGF is especially preferably 10 ng/ml.

In terms of culture conditions, the culture temperature is about 30 to 40°C, preferably about 37°C, although the culture temperature is not limited thereto. The culture is preferably carried out under hypoxic conditions. The term "hypoxic conditions" herein means conditions where the oxygen partial pressure is lower than the oxygen partial pressure in the air (20%). For example, the oxygen partial pressure is from 1% to 15%, and examples of the oxygen partial pressure include 10%, 9%, 8%, 7%, 6%, 5%, 4%, 3%, 2%, and 1%. The oxygen partial pressure is more preferably 5%. The culture is carried out under an atmosphere of air containing CO₂, and the CO₂ concentration is preferably about 2 to 5%.

The culture period is, for example, from 1 day to 7 days. Taking the establishment efficiency of cardiomyocytes into account, examples of the culture period include periods of from 1 day to 5 days, periods of from 1.5 days to 5 days, and periods of from 2 days to 4 days. The culture period in the present invention may be, for example, 1 day, 1.5 days, 2 days, 2.5 days, 3 days, 3.5 days, 4 days, 4.5 days, 5 days, 5.5 days, 6 days, 6.5 days, or 7 days. The culture period may be preferably 2 days.

### <Step of Dissociating Embryoid Bodies in Production Process: Step (3)>

In the present invention, the method for dissociating the embryoid bodies may be the same as the method described for Step (1).

### <Step of Forming Embryoid Bodies by Culturing in Medium Containing VEGF and Wnt Inhibitor and Allowing Reaggregation: Step (4)>

Similarly to Step (1), the culture in this step is preferably suspension culture using a culture vessel whose surface is not artificially treated for the purpose of improving adhesion of cells thereto, or using a culture vessel which is artificially treated such that the adhesion is suppressed.

The medium used in this step can be prepared using, as a basal medium, a medium for animal cell culture, and adding VEGF and a Wnt inhibitor thereto. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, Neurobasal Medium (Life Technologies), and StemPro34 (Invitrogen), and mixed media thereof. The medium may contain serum, or may be serum-free. If necessary, the medium may contain one or more of serum replacements such as albumin, transferrin, Knockout Serum Replacement (KSR) (serum replacement for FBS in ES cell culture), N2 supplement (Invitrogen), B27 supplement (Invitrogen), fatty acids, insulin, collagen precursor, trace elements, 2-mercaptoethanol, and 1-thiolglycerol, and may also contain one or more of substances such as lipids, amino acids, L-glutamine, Glutamax (Invitrogen), non-essential amino acids, vitamins, growth factors, low-molecular-weight compounds, antibiotics, antioxidants, pyruvic acid, buffers, and inorganic salts. A preferred basal medium is StemPro34, which contains transferrin, 1-thiolglycerol, L-glutamine, and ascorbic acid.

In the present invention, the Wnt inhibitor is a substance that inhibits the signaling that proceeds from binding of Wnt to its receptor to accumulation of β-catenin. The Wnt inhibitor is not limited as long as it is a substance that inhibits binding to the receptor Frizzled family, or a substance that promotes degradation of β-catenin. Examples of the Wnt inhibitor include DKK1 protein (for example, in human, NCBI accession No. NM_012242), sclerostin (for example, in human, NCBI accession No. NM_025237), IWR-1 (Merck Millipore), IWP-2 (Sigma-Aldrich), IWP-3 (Sigma-Aldrich), IWP-4 (Sigma-Aldrich), PNU-74654 (Sigma-Aldrich), and XAV939 (Sigma-Aldrich), and their derivatives.

The Wnt inhibitor to be used in this step may be preferably IWP-3 or IWP-4.

The concentration of the Wnt inhibitor such as IWP-3 or IWP-4 in the medium is not limited as long as inhibition of Wnt occurs. The concentration of the Wnt inhibitor is preferably 1 nM to 50 µM. Examples of the concentration of the Wnt inhibitor include, but are not limited to, 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, and 50 µM. The concentration of the Wnt inhibitor is more preferably 1 µM.

The concentration of VEGF to be used in this step is preferably 1 to 100 ng/ml. Examples of the concentration of VEGF include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of VEGF is more preferably 10 ng/ml.

In this step, a BMP inhibitor(s) and/or a TGFβ inhibitor(s) may be further added to the basal medium.

Examples of the BMP inhibitor include protein-based inhibitors such as Chordin, Noggin, and Follistatin; Dorsomorphin (that is, 6-[4-(2-piperidin-1-yl-ethoxy)phenyl]-3-pyridin-4-yl-pyrazolo[1,5-a]pyrimidine) and its derivatives (P. B. Yu et al. (2007), Circulation, 116: II_60; P. B. Yu et al. (2008), Nat. Chem. Biol., 4: 33-41; J. Hao et al. (2008), PLoS ONE, 3(8): e2904); and LDN193189 (that is, 4-(6-(4-(piperazin-1-yl)phenyl)pyrazolo[1,5-a]pyrimidin-3-yl)quinoline).

The TGFβ inhibitor is a substance that inhibits the signaling that proceeds from binding of TGFβ to its receptor to SMAD. Examples of the TGFβ inhibitor include substances that inhibit binding to the receptor ALK family, and substances that inhibit phosphorylation of SMAD by the ALK family. Specific examples of the TGFβ inhibitor include Lefty-1 (e.g., NCBI Accession Nos. NM_010094 (mouse) and NM_020997 (human)); SB431542 and SB202190 (these are described in R. K. Lindemann et al., Mol. Cancer, 2003, 2: 20); SB505124 (GlaxoSmithKline); NPC30345, SD093, SD908, and SD208 (Scios); LY2109761, LY364947, and LY580276 (Lilly Research Laboratories); A83-01 (WO 2009146408); and derivatives thereof.

In terms of culture conditions, the culture temperature is about 30 to 40°C, preferably about 37°C, although the culture temperature is not limited thereto. The culture is preferably carried out under hypoxic conditions. The term "hypoxic conditions" herein means conditions where the oxygen partial pressure is lower than the oxygen partial pressure in the air (20%). For example, the oxygen partial pressure is from 1% to 15%, and examples of the oxygen partial pressure include 10%, 9%, 8%, 7%, 6%, 5%, 4%, 3%, 2%, and 1%. The oxygen partial pressure is more preferably 5%. The culture is carried out under an atmosphere of air containing CO₂, and the CO₂ concentration is preferably about 2 to 5%.

The upper limit of the culture period is not specified since long-term culture does not influence the establishment of cardiomyocytes. The culture is preferably carried out for not less than 4 days. Examples of the culture period include 4 days, 5 days, 6 days, 7 days, 8 days, 9 days, and 10 days. The culture is more preferably carried out for 4 days. This allows the embryoid bodies formed by the reaggregation to differentiate into cardiomyocytes.

### <Step of Culture in Medium Containing VEGF and bFGF: Step (5)>

The medium to be used in this step can be prepared using, as a basal medium, a medium for animal cell culture, and adding VEGF and bFGF thereto. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, Neurobasal Medium (Life Technologies), and StemPro34 (Invitrogen), and mixed media thereof. The medium may contain serum, or may be serum-free. If necessary, the medium may contain one or more of serum replacements such as albumin, transferrin, Knockout Serum Replacement (KSR) (serum replacement for FBS in ES cell culture), N2 supplement (Invitrogen), B27 supplement (Invitrogen), fatty acids, insulin, collagen precursor, trace elements, 2-mercaptoethanol, and 1-thiolglycerol, and may also contain one or more of substances such as lipids, amino acids, L-glutamine, Glutamax (Invitrogen), non-essential amino acids, vitamins, growth factors, low-molecular-weight compounds, antibiotics, antioxidants, pyruvic acid, buffers, and inorganic salts. A preferred basal medium is StemPro34, which contains transferrin, 1-thiolglycerol, L-glutamine, and ascorbic acid.

The concentration of VEGF to be used in this step is preferably 1 to 100 ng/ml. Examples of the concentration of VEGF include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of VEGF is more preferably 10 ng/ml.

The concentration of bFGF to be used in this step is preferably 1 to 100 ng/ml. Examples of the concentration of bFGF include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, and 100 ng/ml. The concentration of bFGF is more preferably 5 ng/ml.

In terms of culture conditions, the culture temperature is about 30 to 40°C, preferably about 37°C, although the culture temperature is not limited thereto. The culture is preferably carried out under hypoxic conditions. The term "hypoxic conditions" herein means conditions where the oxygen partial pressure is lower than the oxygen partial pressure in the air (20%). For example, the oxygen partial pressure is from 1% to 15%, and examples of the oxygen partial pressure include 10%, 9%, 8%, 7%, 6%, 5%, 4%, 3%, 2%, and 1%. The oxygen partial pressure is more preferably 5%. In this step, the oxygen partial pressure may be equivalent to that in the air in the middle of the step. In such cases, although the upper limit is not specified since culturing under hypoxic conditions does not change the efficiency of induction of cardiomyocytes, the culture under hypoxic conditions is preferably carried out for not less than 5 days in the early phase of this step. The culture is carried out under an atmosphere of air containing CO₂, and the CO₂ concentration is preferably about 2 to 5%.

The upper limit of the culture period is not specified since long-term culture does not influence the establishment of cardiomyocytes. The culture is preferably carried out for not less than 12 days. Examples of the culture period include 8 days, 9 days, 10 days, 11 days, 12 days, 13 days, 14 days, 15 days, 16 days, 17 days, 18 days, 19 days, 20 days, 21 days, and 22 days, 23 days, 24 days, 25 days, 26 days, 27 days, 28 days, and periods longer than these. The culture is more preferably carried out for 23 days. By further subjecting the cells obtained in Step (4) to culture according to Step (5), the efficiency of differentiation into cardiomyocytes can be increased.

For increasing purity of the cardiomyocytes finally obtained, the present invention may further include the step of removing cells other than cardiomyocytes during Step (5). This step may be carried out using a method such as the method described in JP 2013-143968 A, but the method is not limited thereto. This step is not limited as long as the culture is carried out under conditions where cardiomyocytes can be separated from cells other than cardiomyocytes. For example, the culture may be carried out under conditions where cardiomyocytes can be separated from cells other than cardiomyocytes by death of the cells other than cardiomyocytes. Examples of the culture conditions where the cells other than cardiomyocytes die include, but are not limited to, culture under low-sugar conditions (for example, at a sugar concentration of not more than 1%, or containing sugar at 55.60 to 111.20 µM) with addition of lactic acid. The culture may be preferably carried out under sugar-free conditions where lactic acid is added. The concentration at which the lactic acid is added is not limited, and is within the range of, for example, 0.1 to 5 mM, preferably 1 mM. The sugar-free culture conditions where lactic acid is added can be achieved by removing sugar from the components of the medium for Step (5) and adding lactic acid thereto. Examples of the method for achieving such conditions include, but are not limited to, a method in which lactic acid is directly added to the medium for Step (5), and a method in which the medium for Step (5) is replaced with fresh medium for Step (5) to which lactic acid is added.

The culture period for the step of removing cells other than cardiomyocytes during Step (5) is preferably not less than 2 days. Examples of the culture period include 2 days, 3 days, 4 days, 5 days, 6 days, 7 days, 8 days, 9 days, and 10 days, and periods longer than these. The culture is more preferably carried out for 4 days.

The timing of starting the culture for removing cells other than cardiomyocytes is preferably Day 10 after the beginning of Step (5), or later. Examples of the time include Day 10, Day 11, Day 12, Day 13, Day 14, Day 15, Day 16, Day 17, Day 18, Day 19, Day 20, Day 21, Day 22, Day 23, Day 24, Day 25, or Day 26, or a time later than these. The culture is more preferably started on Day 16.

### <Drug Reactivity Test>

In the present invention, whether a drug candidate substance has cardiotoxicity or not can be investigated using mature cardiomyocytes extracted using a marker(s) of the present invention. The test for cardiotoxicity can be carried out by an arbitrary method based on a common technical knowledge in this field. The test in the present invention can be carried out based on, for example, an electrophysiological method, analysis of expression of a toxicity marker gene(s), and/or the like. Examples of the electrophysiological method include, but are not limited to, the patch clamp test. Examples of the toxicity marker gene(s) used for the analysis of expression of a toxicity marker gene(s) include ABHD2, CCR1, GJA1, NEXN, PSMD7, TGFB2, VIM, ABRA, CD14, GPM6A, NFIB, PUM2, THRAP3, WIPI1, ACTA1, CFD, HAMP, PDK4, PVR, TIAM1, ZNF148, AIFM1, COL15A1, HSPA2, PKN2, RBM3, TIMP1, ZNF23, AK3, COL3A1, HSPH1, PLA2G4A, REG3G, TUBB6, ASH1L, CREM, IFT20, PLAU, RND1, TXNIP, ATP5J, CSNK2A2, IGFBP5, PLN, RPS6KB1, UBA5, BCAT1, DUSP8, IL6, POSTN SERPINE1 UBXN2A, BGN, EGR1, ITPR2, PPBP, SIK1, UCK2, BSN, FCGR2B, KBTBD10, PPP1R14C, SLC4A3, UCP1, BTG2, FHL1, KBTBD5, PRKAB2, SPP1, VCAN, CCL7, FOSL1, MCM6, PSMA2, TCF4, and VEGFA. Measurement and analysis of the toxicity marker gene(s) can be carried out by targeting the gene(s) using, for example, TruSeq Target RNA Expression Analysis - Cardiotoxity Panel (Illumina, Inc.; Catalog ID: RT-202-1009).

In the present invention, examples of the cardiotoxicity of the drug candidate substance include drug-induced arrhythmia. Examples of the arrhythmia include tachycardia, bradycardia, extrasystole, and QT prolongation. The arrhythmia detected in the method of the present invention is preferably QT prolongation. Thus, the cardiotoxicity to be tested in the present invention can be said to be drug-induced QT prolongation. Since QT prolongation is caused by inhibition of potassium channels, the method of the present invention can also be said to be a method for testing the potassium channel-inhibiting property of the drug candidate substance that causes arrhythmia.

In the present invention, the QT prolongation can be confirmed by confirming continuation of the change in the potential of mature cardiomyocytes obtained by a patch clamp test. In the present invention, the continuation of the change in the potential of mature cardiomyocytes means the interval between shifting of the resting membrane potential toward the positive potential side and recovery of the resting membrane potential.

A drug candidate substance can be identified as a drug having no cardiotoxicity when mature cardiomyocytes brought into contact with the compound can maintain a normal function, preferably an electrophysiologically normal function. On the other hand, a drug candidate substance can be identified as a drug having cardiotoxicity when the period of continuation of the change in the potential in mature cardiomyocytes brought into contact with the drug is prolonged based on comparison between the period of continuation of the change in the potential in mature cardiomyocytes that were brought into contact with the drug candidate substance and the period of continuation of the change in the potential in mature cardiomyocytes that were not brought into contact with the drug candidate substance.

In the present invention, the drug candidate substance is not limited as long as it is a substance assumed to be effective for a disease(s). Examples of the drug candidate substance include cell extracts, cell culture supernatants, microbial fermentation products, extracts derived from marine organisms, plant extracts, purified proteins and crude proteins, peptides, nonpeptide compounds, synthetic low molecular compounds, and naturally occurring compounds. The disease to be treated with the drug candidate substance in the present invention is not limited to cardiac diseases.

The cells used in the drug reactivity test in the present invention may be either single cells, or a cell cluster or a cell sheet. In cases where the cells used are a cell cluster, the cell cluster is preferably constituted only by mature cardiomyocytes, but may also be a mixture containing a small amount of other cells as well as mature cardiomyocytes.

In the present invention, the cell sheet can be obtained by subjecting mature cardiomyocytes obtained by the method of the present invention described above to culture using a culture equipment coated with a temperature-responsive polymer prepared by polymerizing (meth)acrylamide compound, *N*-(or *N,N*-di)alkyl-substituted (meth)acrylamide derivative (JP 2010-255001 A), or vinyl ether derivative. For example, the culture equipment can be purchased from CellSeed Inc. as UpCell.

### <Method for Extraction or Detection of Cardiomyocytes>

The reagent to be used for the extraction or detection of cardiomyocytes from a cell population containing cardiomyocytes is not limited as long as it has specific affinity to CORIN, NCAM1, CRYAB, HBEGF, DMD, ATPIF1, CAV2, ITGAV, DCBLD2, CLIC4, BMPR2, CTSB, TMEM123, USP14, and/or MIR761. Examples of the reagent that may be used include antibodies, aptamers, peptides, and specifically-recognizing compounds. The reagent is preferably an antibody or a fragment thereof.

For investigation of gene expression of these markers, primers and probes that hybridize with these marker genes may be used.

In the present invention, the antibody may be either a polyclonal or monoclonal antibody. These antibodies can be prepared using techniques well known to those skilled in the art (Current protocols in Molecular Biology edit. Ausubel et al. (1987) Publish. John Wiley and Sons. Sections 11.12-11.13). More specifically, in cases where the antibody in the present invention is a polyclonal antibody, the protein CORIN, NCAM1, CRYAB, HBEGF, DMD, ATPIF1, CAV2, ITGAV, DCBLD2, CLIC4, BMPR2, CTSB, TMEM123, USP14, or MIR761 which was expressed in *E. coli* or the like and purified according to a conventional method, or a synthetic oligopeptide having a partial amino acid sequence of the protein, may be used to immunize a non-human animal such as a rabbit, followed by obtaining the antibody from serum of the immunized animal according to a conventional method. In cases where the antibody is a monoclonal antibody, the monoclonal antibody can be obtained from hybridoma cells prepared by cell fusion of spleen cells obtained from the above-described immunized non-human animal with myeloma cells (Current protocols in Molecular Biology edit. Ausubel et al. (1987) Publish. John Wiley and Sons. Sections 11.4-11.11). Examples of the fragment of the antibody include a part of the antibody (e.g., Fab fragment) and a synthetic antibody fragment (e.g., single-stranded Fv fragment, "ScFv"). Antibody fragments such as Fab and F(ab)₂ fragments can also be prepared by well-known methods in genetic engineering. In cases where the marker is a membrane protein, the antibody is preferably an antibody against the extracellular domain. Examples of the antibody include the antibody against CORIN disclosed in WO 2006/009241.

In order to distinguish and separate cells to which the reagent having affinity is bound, the reagent may be bound or conjugated to a substance which can be detected, such as a fluorescent label, radioactive label, chemiluminescent label, enzyme, biotin, or streptavidin, or to a substance which enables isolation and extraction, such as protein A, protein G, beads, or magnetic beads.

The reagent having affinity may also be indirectly labeled. The labeling can be carried out by various methods known to those skilled in the art, and examples of the methods include a method wherein a preliminarily labeled antibody (secondary antibody) that specifically binds to an antibody against CORIN, NCAM1, CRYAB, HBEGF, DMD, ATPIF1, CAV2, ITGAV, DCBLD2, CLIC4, BMPR2, CTSB, TMEM123, USP14, or MIR761 is used.

Examples of the method for detecting cardiomyocytes include flow cytometry and a method wherein the cells are isolated and purified followed by detection of the cells (using, for example, a protein chip).

Examples of the method for extracting cardiomyocytes include a method wherein a large particle is conjugated to the reagent having affinity to cause precipitation, a method wherein cells are sorted by the magnetism using magnetic beads (e.g., MACS), a method wherein a fluorescent label is used to employ a cell sorter, and a method wherein a carrier to which an antibody or the like is immobilized (e.g., cell-concentrating column) is used.

In the present invention, mature cardiomyocytes can be extracted from a cell population containing cardiomyocytes by selectively collecting cells that are positive for at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761. In the present invention, the selective collection (extraction) of cells that are positive for a marker(s) may be collection (extraction) of all cells that are positive for the markers), or may be collection (extraction) of cells showing not less than a predetermined level(s) of expression of the marker(s). For example, cells in which the expression level(s) of the marker(s) is/are ranked in the top 50%, ranked in the top 40%, ranked in the top 33%, ranked in the top 30%, ranked in the top 20%, or ranked in the top 10% may be selectively collected from a cell population containing a cardiomyocyte.

The present invention also provides a cardiomyocyte extraction kit containing a reagent which detects at least one selected from the group consisting of CORIN, CRYAB, HBEGF, DMD, ATPIF1, CAV2, ITGAV, DCBLD2, CLIC4, BMPR2, CTSB, TMEM123, USP14, and MIR761. The present invention also provides a cardiomyocyte extraction kit containing a reagent for detection of NCAM1. The detection reagent contained in this extraction kit is as described above. The extraction kit in the present invention may contain an instruction in which a method for using the detection reagent is described, together with a detection reagent for CORIN, NCAM1, CRYAB, HBEGF, DMD, ATPIF1, CAV2, ITGAV, DCBLD2, CLIC4, BMPR2, CTSB, TMEM123, USP14, and/or MIR761.

### EXAMPLES

The present invention is described below more concretely by way of Examples. However, needless to say, the present invention is not limited to these.

### Example 1

### <Identification of Mature Cardiomyocyte Markers>

### Pluripotent Stem Cells

An experiment was carried out using, as pluripotent stem cells, the following iPS cell lines, that is, the 201B7 line and the 201B7 line having the MYH6-GFP reporter (hereinafter referred to as MYH6-GFP reporter line). The 201B7 line was prepared by the method described in Takahashi K, et al. Cell. 131: 861-72, 2007. The MYH6-GFP reporter line was prepared by introducing a vector in which an EGFP cassette is operably linked downstream of an MYH6 (myosin heavy chain 6) promoter, into the 201B7 line using the PiggyBac transposon system (System Biosciences, Inc.). Culture of the 201B7 line and the MYH6-GFP reporter line was carried out by a conventional method (Takahashi K, et al. Cell. 131: 861-72, 2007 and Nakagawa M, et al. Nat Biotechnol. 26: 101-6, 2008).

### Cardiomyocyte Induction Method

After treatment of the MYH6-GFP reporter line with CTK solution (ReproCELL) for 2 minutes, the solution was removed. Subsequently, the cells were treated with Accumax (Innovative Cell Technologies) for 5 minutes, and dissociated into single cells by pipetting. The medium was removed by centrifugation, and the obtained cells were plated on a low-adhesion 96-well dish (Corning) at 2500 cells/well. Culture was carried out at 37°C under 5% oxygen in STEMPRO 34 supplemented with 1% L-glutamine, 150 µg/mL transferrin, 50 µg/mL ascorbic acid (Sigma), 3.9×10⁻³% MTG, 10 µM Rock inhibitor, 2 ng/mL BMP4 (R&D), and 0.50% Matrigel (Growth Factor Reduced), to allow formation of embryoid bodies (hereinafter referred to as EBs) (Day 0).

On the next day (Day 1), an equal volume of STEMPRO 34 supplemented with 1% L-glutamine, 150 µg/mL transferrin, 50 µg/mL ascorbic acid, 3.9×10⁻³% MTG, 18 ng/mL BMP4, 10 ng/mL bFGF, and 12 ng/mL activin A was added to the 96-well plate during the culture of EBs, and culture was carried out at 37°C under 5% oxygen for additional 2 days.

Subsequently (Day 3), the obtained EBs were allowed to naturally precipitate, and the medium was removed. Accumax was added to the EBs, and, after 5 minutes of treatment, the EBs were dissociated into single cells by pipetting. After addition of 5 ml of IMDM (Invitrogen), the medium was removed by centrifugation. The obtained cells were plated on a low-adhesion 96-well dish (Corning) at 10,000 cells/well, and EBs were allowed to form in STEMPRO 34 supplemented with 1% L-glutamine, 150 µg/mL transferrin, 50 ng/mL ascorbic acid, 3.9×10⁻³% MTG, 10 ng/mL VEGF, and 1 µM IWP-3, followed by performing culture at 37°C under 5% oxygen for 4 days.

Subsequently (Day 7), the obtained EBs were recovered, and transferred to a 24-well dish such that the number of EBs per well did not exceed 10. Culture was carried out for 5 days at 37°C under 5% oxygen in STEMPRO 34 supplemented with 1% L-glutamine, 150 µg/mL transferrin, 50 ng/mL ascorbic acid, 3.9×10⁻³% MTG, 10 ng/mL VEGF, and 5 ng/mL bFGF. During this culture, the medium was replaced every other day with the same fresh medium.

Subsequently (Day 12), the dish was transferred to an incubator with normal oxygen concentration, and culture was carried out for 18 days (Day 30). During this culture, the medium was replaced every other day with the same fresh medium.

### Identification of Mature Cardiomyocyte Markers

By the cardiomyocyte induction method described above, differentiation of the MYH6-GFP reporter line was induced until Day 9, Day 21, or Day 30. The cells on Day 9, Day 21, and Day 30, and the MYH6-GFP reporter line before the differentiation induction were subjected to single-cell RNA seq analysis, and the average for each cell population was calculated to provide bulk data. Subsequently, based on the following four criteria, genes satisfying all criteria were identified.
1. Cardiomyocytes on Days 21 and 30 > Cardiomyocytes on Day 9; Fold Change (FC) >2.0
2. Cardiomyocytes on Days 21 and 30 > iPS cells (MYH6-GFP reporter line); FC >5.0
3. Adult heart > fetal heart; FC >2.0
4. Adult heart > other tissues (liver, kidney, and brain); FC>5.0

As a result, 39 genes were identified as genes satisfying all criteria. The weighted average of their gene expression was calculated and expressed as a numerical value to define a maturity index.

Subsequently, for each of a total of 46 samples on Day 21 and Day 30, sorting was carried out according to the maturity index in the order from high to low values (in the order of maturity). The correlation coefficient for the maturity index was calculated for each of all genes, and the surface marker genes were listed in the order from high to low correlation coefficients. From the top 15 genes that were likely to be useful as mature cardiomyocyte markers, CORIN was selected to provide an example, and subjected to the experiments in Example 2 and later Examples.

The top 15 genes were as shown in the following Table 2.

**[Table 2]**

| Top 15 genes | Correlation coefficient |
|---|---|
| CRYAB | 6.04E-01 |
| NCAM1 | 5.70E-01 |
| CORIN | 4.39E-01 |
| HBEGF | 4.33E-01 |
| DMD | 3.95E-01 |
| ATPIF1 | 3.72E-01 |
| CAV2 | 3.63E-01 |
| ITGAV | 3.40E-01 |
| DCBLD2 | 3.07E-01 |
| CLIC4 | 3.01E-01 |
| BMPR2 | 2.83E-01 |
| CTSB | 2.75E-01 |
| TMEM123 | 2.72E-01 |
| USP14 | 2.54E-01 |
| MIR761 | 2.43E-01 |

### Example 2

### Extraction of Mature Cardiomyocytes Using CORIN

Whether or not CORIN, which was identified as a novel mature cardiomyocyte marker in Example 1, is actually effective for extraction of mature cardiomyocytes was investigated in detail. Briefly, the MYH6-GFP reporter line on Day 30 differentiated by the cardiomyocyte induction method in Example 1 was subjected to FACS analysis using as indices GFP and CORIN, which substitute expression of a known cardiomyocyte marker MYH6. The FACS analysis was carried out using a FACS Arial II cell sorter (BD Biosciences). As a result, cells in which the expression levels of both GFP and CORIN are high (High group) and cells in which the expression level of GFP is high, but the expression level of CORIN is low (Low group) were found. The High group and the Low group were further sorted using a FACS Arial II cell sorter (Fig. 1(A)). The High group was sorted as the cells with the expression levels of CORIN ranked in the top 33%, and the Low group was sorted as the cells with the expression levels of CORIN ranked in the bottom 33%.

The High group and the Low group obtained were subjected to measurement of the relative expression levels of cardiomyocyte markers (MYH7, MYL2, MYL7, TCAP, SCN5A, and RYR2) to investigate maturity of cardiomyocytes. The measurement of the expression levels was carried out by quantitative RT-PCR. Briefly, mRNA was isolated from the cells using an RNeasy Mini Kit (Qiagen). Using 1 µg of the obtained total RNA as a template, cDNA was synthesized by reverse transcription using Superscript III reverse transcriptase (Invitrogen). In the quantitative RT-PCR analysis, Power SYBR Green qPCR mastermix (Invitrogen) was used, and the measurement was carried out with the StepOne real-time PCR system (ABI). As a result, it was found that any of the investigated cardiomyocyte markers shows a higher expression level in the High group than in the Low group (Fig. 1(B)). This suggests that the High group is a group of relatively mature cardiomyocytes.

Subsequently, an experiment was carried out for investigating the electrophysiological function in the High group and the Low group obtained. Briefly, the cells on Day 30 were sorted into the High group and the Low group by FACS in the same manner as described above, and then cultured for 5 days, followed by performing patch clamp analysis. The patch clamp analysis was carried out using an Axopatch 200B amplifier (Molecular Devices, Sunnyvale, CA) according to description in Anna L. Lahti et al., Dis Model Mech., 5: 220-230 (2012) and Djamel Lebeche, et al., Circulation., 110: 3435-3443 (2004) (Fig. 2(A)). As a result, the rest potential (resting membrane potential) shifted more to the negative side in the High group than in the Low group (Fig. 2(B)), and the peak voltage and the amplitude were found to show higher absolute values in the High group than in the Low group (Fig. 2(C) and (D)). The more the resting membrane potential shifts to the negative side, the more mature the cardiomyocytes are suggested to be. The more the peak voltage and the amplitude shift to the positive side, the more mature the cardiomyocytes are suggested to be. Thus, the above results suggest that the High group has a function closer to that of mature cardiomyocytes in the living body.

### Example 3

### Extraction of Mature Cardiomyocytes Using CORIN

Whether or not CORIN, which was identified as a novel mature cardiomyocyte marker in Example 1, allows extraction of mature cardiomyocytes from cardiomyocytes obtained by a partially modified cardiomyocyte induction method was studied. Briefly, the partial modification was inclusion of a step of culture in a glucose-free medium to which lactic acid was added, on Days 23 to 27 in the cardiomyocyte induction method in Example 1. The MYH6-GFP reporter line on Day 30 was subjected to FACS analysis using a known cardiomyocyte marker (TNT) as an index. As a result, 94.7% of the cells were positive for TNT, so that their differentiation into cardiomyocytes was suggested (Fig. 3(B)). The FACS analysis was carried out using a FACS Arial II cell sorter (BD Biosciences) in the same manner as in Example 2.

Subsequently, the TNT-positive cells were subjected to FACS analysis using CORIN as an index. As a result, cells in which the expression level of CORIN is high (CORIN-High group) and cells in which the expression level of CORIN is low (CORIN-Low group) were found. The CORIN-High group and the CORIN-Low group could be further sorted using a FACS Arial II cell sorter (Fig. 3(C)). The CORIN-High group was sorted as the cells with the expression levels of CORIN ranked in the top 33%, and the CORIN-Low group was sorted as the cells with the expression levels of CORIN ranked in the bottom 33%.

Subsequently, the CORIN-High group and the CORIN-Low group were subjected to measurement of the relative expression levels of cardiomyocyte markers (MYH7, MYL2, MYL7, TCAP, SCN5A, and RYR2) to investigate maturity of cardiomyocytes. The measurement of the expression levels was carried out by RT-PCR by the same method as described above. As a result, it was found that any of the investigated cardiomyocyte markers shows a higher expression level in the CORIN-High group than in the CORIN-Low group (Fig. 3(D)). This suggests that the CORIN-High group is a group of relatively mature cardiomyocytes.

### Example 4

### Functional Analysis of Mature Cardiomyocytes Extracted Using CORIN

### <Morphological Analysis>

Cardiomyocytes on Day 30 after the differentiation induction from the iPS cells were plated again on a two-dimensional culture dish, and then observed using a microscope. As a result, as shown in Fig. 4, the CORIN-High group was found to show larger cell sizes than the CORIN-Low group.

### <Structural Analysis>

Cardiomyocytes on Day 30 after the differentiation induction from the iPS cells were plated again on a fibronectin-coated dish, and cell sections were prepared 3 days thereafter, followed by analysis using a transmission electron microscope (H-7650, Hitachi). As a result, as shown in Figs. 5 and 6, the CORIN-High group showed a clearly arranged sarcomere structure with a sarcomere width remarkably longer than that of the CORIN-Low group.

### <Functional Analysis>

For investigation of the mitochondrial function, cardiomyocytes (20,000 cells) on Day 30 after the differentiation induction from iPS cells were plated on an assay well (Seahorse Bioscience) coated with fibronectin. Three days after the plating, the oxygen consumption rate (OCR) was measured with an XF24 extracellular flux analyzer manufactured by Seahorse Bioscience. As a result, as shown in Fig. 7, the CORIN-High group was found to show a higher oxygen consumption rate as well as higher activity of the electron transfer system.

Based on these results, the fact that mature cardiomyocytes can be concentrated using as an index high expression of CORIN could be confirmed based not only on the transcription level, but also on the structural, electrophysiological, and metabolic studies.

### INDUSTRIAL APPLICABILITY

Usually, a cardiomyocyte population obtained by differentiation induction from pluripotent stem cells or the like contains cells showing various degrees of maturity. By the present invention, cells in which cardiomyocytes with higher degrees of maturity that are closer to the cardiomyocytes present in the living body are concentrated can be provided from such a cell population. The more uniformly purified mature cardiomyocytes obtained by the present invention can be used for drug toxicity tests and cardiac disease model studies that are more accurate than conventional tests and studies. The cells are therefore useful for reduction of the development periods for drugs, and search for novel disease targets.

## Claims

1. A method for producing cardiomyocytes, comprising extracting mature cardiomyocytes from a cell population containing cardiomyocytes using, as an index, positivity of at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.

2. The method according to claim 1, wherein said marker is CORIN.

3. The method according to claim 1 or 2, wherein cells in which the expression level(s) of said marker(s) is/are ranked in the top 50% are extracted as mature cardiomyocytes from the cells contained in the cell population containing cardiomyocytes.

4. The method according to claim 3, wherein cells in which the expression level(s) of said marker(s) is/are ranked in the top 33% are extracted as mature cardiomyocytes from the cells contained in the cell population containing cardiomyocytes.

5. The method according to any one of claims 1 to 4, wherein said cardiomyocytes are human cardiomyocytes.

6. The method according to any one of claims 1 to 5, wherein said cell population containing cardiomyocytes is a cell population containing cardiomyocytes obtained by differentiation induction from pluripotent stem cells.

7. The method according to claim 6, wherein said pluripotent stem cells are ES cells or iPS cells.

8. The method according to claim 6 or 7, wherein said differentiation induction into cardiomyocytes comprises forming an embryoid body/bodies.

9. The method according to claim 8, wherein said differentiation induction into cardiomyocytes comprises culturing an embryoid body/bodies in a medium containing a cytokine.

10. The method according to claim 9, wherein said cytokine is at least one cytokine selected from the group consisting of activin A, BMP4, b-FGF, VEGF, and Wnt inhibitors.

11. The method according to claim 10, wherein said differentiation induction into cardiomyocytes comprises:
(1) forming an embryoid body/bodies from pluripotent stem cells;
(2) culturing the embryoid body/bodies obtained in Step (1) in a medium containing activin A, BMP4, and bFGF;
(3) dissociating the embryoid body/bodies obtained in Step (2);
(4) culturing the cells obtained in Step (3) in a medium containing VEGF and a Wnt inhibitor, to allow reaggregation of the cells into an embryoid body/bodies; and
(5) culturing the embryoid body/bodies obtained in Step (4) in a medium containing VEGF and bFGF.

12. The method according to claim 11, wherein said Wnt inhibitor is IWP-3 or IWP-4.

13. The method according to claim 11 or 12, wherein Step (5) in the differentiation induction into cardiomyocytes includes a culture period when the cells are cultured in a medium which contains lactic acid but does not contain glucose.

14. A method for testing cardiotoxicity of a drug candidate substance, comprising:
(A) bringing mature cardiomyocytes prepared by the method according to any one of claims 1 to 13 into contact with a candidate substance; and
(B) identifying said candidate substance as a drug having no cardiotoxicity in a case where the cardiomyocytes maintains a normal function after the contact in said step (A).

15. The method according to claim 14, wherein said normal function of the cardiomyocytes is an electrophysiologically normal function.

16. A mature cardiomyocyte extraction kit comprising a reagent which detects at least one selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.

17. The kit according to claim 16, comprising a reagent for detection of CORIN.

18. The kit according to claim 16 or 17, wherein said cardiomyocyte is a human cardiomyocyte.

19. A method for producing cardiomyocytes, comprising:
obtaining a cell population containing cardiomyocytes from pluripotent stem cells by the following steps (1) to (5):
(1) forming an embryoid body/bodies from pluripotent stem cells;
(2) culturing the embryoid body/bodies obtained in Step (1) in a medium containing activin A, BMP4, and bFGF;
(3) dissociating the embryoid body/bodies obtained in Step (2);
(4) culturing the cells obtained in Step (3) in a medium containing VEGF and a Wnt inhibitor, to allow reaggregation of the cells into an embryoid body/bodies; and,
(5) culturing the embryoid body/bodies obtained in Step (4) in a medium containing VEGF and bFGF; and
extracting mature cardiomyocytes from the obtained cell population containing cardiomyocytes using, as an index, positivity of at least one marker selected from the group consisting of CORIN, NCAM1, CRYAB, HBEGF, DMD, ATPIF1, CAV2, ITGAV, DCBLD2, CLIC4, BMPR2, CTSB, TMEM123, USP14, and MIR761.
